# EUROPÄISCHE PATENTANMELDUNG

(11) **EP 1 348 376 A2**
(43) Veröffentlichungstag der Anmeldung: **01.10.2003**
(21) Anmeldenummer: 03006036.2
(22) Anmeldetag: 19.03.2003
(51) Int. Cl.: A61B 6/08, A61B 5/113

(54) **Vorrichtung zur berührungsfreien Messung eines Abstandes bei der Bestrahlung des menschlichen Körpers**

(30) Priorität: 30.03.2002 DE 20205049 U
(71) Anmelder: LAP GmbH Laser Applikationen, D-21337 Lüneburg (DE)
(72) Erfinder: Roeckseisen, Armin, Dr., 21365 Adendorf (DE)
(74) Vertreter: Patentanwälte Hauck, Graalfs, Wehnert, Döring, Siemons, Schildberg

(57) **Zusammenfassung**

Vorrichtung zur berührungsfreien Messung eines Abstands von einem menschlichen Körper bei der Bestrahlung, mit einem verfahrbaren Strahlungskopf, der einen Behandlungsstrahl auf das zu behandelnde Gewebegebiet richtet, einer an dem Strahlungskopf in einem definierten Abstand zu dem Strahl vorgesehenen Halterung für eine Abstandsmesseinrichtung, die in einer Messstellung einen Laserstrahl auf einen Durchtrittspunkt des Behandlungsstrahls auf der Haut gerichtet ist und den Abstand zu dem Durchtrittspunkt misst, und einer Auswerteeinrichtung, die aus dem gemessenen Abstandswert den Abstand vom Fokus des Behandlungsstrahls zu dem Durchtrittspunkt berechnet.

## Beschreibung

Die Erfindung betrifft eine Vorrichtung zur berührungsfreien Messung eines Abstandes bei der Bestrahlung des menschlichen Körpers, insbesondere den Abstand von einem Fokus des Behandlungsstrahls zu der Haut des Patienten.

Bei der Bestrahlung kommt es darauf an, ein zu behandelndes Gewebegebiet, beispielsweise mit einem Tumor, mit Behandlungsstrahlen zu treffen und gleichzeitig das umliegende Gewebe so wenig wie möglich zu belasten. Der Behandlungsstrahl ist ab der Strahlungsquelle divergent. Um das Behandlungsgebiet einzuschränken, wird der Behandlungsstrahl durch feste Blenden aus Metallegierung, so genannte Blöcke, oder durch Lamellenblenden, so genannte Multileaf-Kollimatoren, verformt. Hierdurch kann das zur Bestrahlung vorgesehene Gewebegebiet in seiner vielfältigen dreidimensionalen Form gut durch den Behandlungsstrahl erfasst werden. Durch Bewegung der Gantry mit dem Bestrahlungskopf und eine zusätzliche Bewegung des Patiententisches können beliebig geformte Behandlungsgebiete mit Behandlungsstrahlen bestrahlt werden. Hierbei ist es möglich, mit variabler Strahlenleistung aus beliebigen Richtungen das Gewebegebiet zu bestrahlen. Diese Technik der Bestrahlung wird als "Intensitätsmodulierte Strahlentherapie" (IMRT = Intensity modulated radiotherapy) bezeichnet.

Wichtige Voraussetzung für diese Bestrahlungstechnik ist jedoch, dass der Tumor sich in der von der Steuerung der Strahlentherapie vorgesehenen Lage befindet. Hierzu sind verschiedenste Lagerungs- und Fixationstechniken bekannt. Jedoch kann bereits die Atembewegung des Patienten die Lage des zu behandelnden Gewebegebiets relativ zu dem Strahlungskopf und damit zum Fokus des Behandlungsstrahls verändern.

Linearbeschleuniger zur Bestrahlung sind durchweg mit einem Telemeter ausgerüstet. Hierbei handelt es sich um ein optisches System zur Messung des Fokus/Haut-Abstandes (SSD). Der SSD wird entlang dem Behandlungsstrahl für den aktuell eingestellten Gantrywinkel gemessen. Der Messwert des Telemeters muss durch den menschlichen Beobachter abgelesen werden, daher muss eine Strahlenbehandlung zur Bestimmung des Messwerts unterbrochen werden.

Der Erfindung liegt die Aufgabe zugrunde, eine Vorrichtung zur berührungsfreien Messung eines Abstandes bei der Bestrahlung zu schaffen, die mit einfachen Mitteln eine zuverlässige Messung des SSD gestattet.

Erfindungsgemäß wird die Aufgabe durch eine Vorrichtung mit den Merkmalen aus Anspruch 1 gelöst. Vorteilhafte Ausgestaltungen bilden den Gegenstand der Unteransprüche.

Die erfindungsgemäße Vorrichtung besitzt einen verfahrbaren Strahlungskopf, der einen Behandlungsstrahl auf ein zu behandelndes Gewebegebiet richtet. An dem Strahlungskopf ist in einem definierten Abstand zu dem Behandlungsstrahl eine Halterung für eine Abstandsmesseinrichtung vorgesehen. Die Abstandsmesseinrichtung richtet in einer Messstellung einen Laserstrahl auf den Durchtrittspunkt des Behandlungsstrahls auf der Haut, insbesondere auf das Zentrum des Behandlungsstrahls. Die Abstandsmesseinrichtung misst den Abstand von einem definierten Referenzpunkt der Abstandsmesseinrichtung zu dem auf die Haut projizierten Durchtrittspunkt nach dem Prinzip der Lasertriangulation. Zusätzlich besitzt die erfindungsgemäße Vorrichtung eine Auswerteeinrichtung, die aus dem gemessenen Abstandswert den Abstand vom Fokus des Behandlungsstrahls zu dem Durchtrittspunkt berechnet. Bei der erfindungsgemäßen Vorrichtung wird berührungslos nach dem Prinzip der Lasertriangulation der Abstand von Haut und Fokus bestimmt. Die Abstandsmesseinrichtung ist hierbei außerhalb der Achse des Behandlungsstrahls angeordnet und wird auf den Durchtrittspunkt des Behandlungsstrahls auf der Haut gerichtet. Zur Bestimmung des Abstandes entlang dem Behandlungsstrahl werden die Abstandswerte mit Hilfe der Auswerteeinrichtung umgerechnet. Als besonders vorteilhaft an der erfindungsgemäßen Vorrichtung erweist es sich, dass im Behandlungsraum keine Bedienperson zum Ablesen der Abstandswerte benötigt wird. Die Strahlenbehandlung, bei der keine Bedienperson im Raum sein darf, kann somit insgesamt schneller durchgeführt werden, da keine Bedienperson die Messwerte optisch ablesen muss.

Die Messeinrichtung misst den Abstand zu dem auf die Haut projizierten Laserpunkt nach dem Prinzip der Lasertriangulation. Bei der Lasertriangulation wird ein Laserstrahl ausgesendet. Trifft der Laserstrahl auf ein Hindernis, so erscheint ein Laserpunkt, dessen Lage durch einen lichtempfindlichen Sensor aufgezeichnet wird. Sensor und Laserquelle stehen in einer fest definierten geometrischen Beziehung zueinander, sodass aus der Lage bzw. dem Winkel, unter dem der Laserpunkt beobachtet wird, der Abstand berechnet werden kann.

Um die Abstandseinrichtung in ihre Stellung zu bringen, ist die Halterung mit einer Schwenkachse versehen, wobei die Schwenkachse senkrecht zu dem Behandlungsstrahl steht. Die Schwenkachse der Halterung ist so ausgerichtet, dass der Laserstrahl in jeder Stellung den Behandlungsstrahl schneidet. Die definierte Lage der Schwenkachse relativ zu dem Behandlungsstrahl ermöglicht es, dass zur Berechnung des Abstandes entlang dem Behandlungsstrahl die Winkelstellung der Halterung nicht erfasst werden muss.

Bevorzugt kommt hier das Meßprinzip der Off-axis Triangulation zum Einsatz, wie es für die Anmelderin in US 6,088,106 geschützt ist.

In einer bevorzugten Ausgestaltung, insbesondere bei dem Einsatz der intensitätsmodulierten Strahlentherapie, sind Sollwerte für den Abstand vorgegeben. Die Auswerteeinrichtung schaltet bei Abweichungen des gemessenen Abstandes von den vorbestimmten Sollwerten die Strahlenquelle ab oder vermindert deren Qualität. Auf diese Art und Weise wird sichergestellt, dass lediglich die berechnete Menge an Strahlung zu dem vorgesehenen Gewebegebiet gelangt.

In bevorzugten Weiterführungen der erfindungsgemäßen Vorrichtung wird der gemessene Abstandswert als Führungsgröße für eine Steuerung der Position des Patiententisches und/oder der Strahlungsintensität eingesetzt.

Eine bevorzugte Ausgestaltung der erfindungsgemäßen Vorrichtung wird nachfolgend in einem Ausführungsbeispiel näher erläutert.

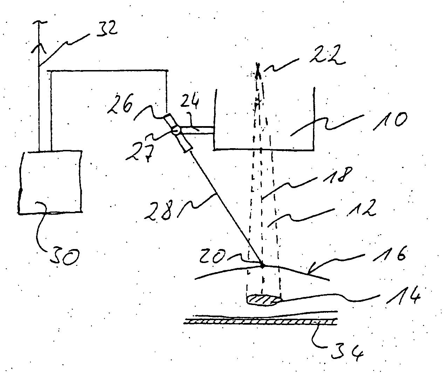

Die einzige Figur zeigt einen Bestrahlungskopf 10, der einen divergierenden Behandlungsstrahl 12 auf ein zu behandelndes Gewebegebiet 14 richtet. Die Berechnung und Formung des Behandlungsstrahls 12 entsprechend dem Tumorbereich ist bekannt.

Für die intensitätsmodulierte Strahlentherapie ist der Fokus/Haut-Abstand (SSD = Source/Skin Distance) eine bestimmte Größe, insbesondere bei der IMRT. Der Behandlungsstrahl 12 trifft in einem Bereich auf die Haut 16. Für die intensitätsmodulierte Strahlentherapie wird der Abstand entlang des Mittelstrahls 18 betrachtet. Der SSD bestimmt sich als Strecke von einem Referenzpunkt des Behandlungsstrahls 12 zu dem Durchstoßpunkt 20 des Mittenstrahls. In der Figur ist ein gedachter Fokus 22 beispielhaft eingezeichnet.

An dem Strahlungskopf 10 ist mit einer Halterung 24 eine Abstandsmesseinrichtung 26 schwenkbar gelagert. Die Abstandsmesseinrichtung 26 arbeitet nach dem Prinzip der Lasertriangulation. Der Laserstrahl 28 ist zur Messung auf den Durchstoßpunkt 20 gerichtet. In dem Durchstoßpunkt 20 entsteht ein Laserpunkt, der von der Abstandsmesseinrichtung 26 unter einem entsprechenden Winkel beobachtet wird. Hebt und senkt sich nun der Punkt 20, so ändert sich der Beobachtungswinkel und der Abstand entlang dem Laserstrahl 28.

Eine schematisch dargestellte Auswerteeinrichtung wertet den gemessenen Abstand aus und berechnet aus diesem den SSD. Der SSD wird über eine Leitung 32 an eine Steuerung weitergeleitet.

Die Abstandsmesseinrichtung 26 ist um die senkrecht zur Papierebene der Zeichnung stehende Achse 27 schwenkbar. Die Schwenkachse 27 ist so ausgerichtet, dass unter jedem Winkel der Laserstrahl 28 den Behandlungsstrahl 18 schneidet.

Der gemessene Abstandswert kann von der Steuerung der Strahlungsquelle zur Modulation der Strahlungsintensität eingesetzt werden. Ebenfalls kann der Patiententisch 34 in seiner Position abhängig von dem gemessenen Abstandswert eingestellt werden.

## Patentansprüche

1. Vorrichtung zur berührungsfreien Messung eines Abstands von einem menschlichen Körper bei der Bestrahlung, mit:
- einem verfahrbaren Strahlungskopf (10), der einen Behandlungsstrahl (12, 18) auf das zu behandelnde Gewebegebiet (14) richtet,
- einer an dem Strahlungskopf (10) in einem definierten Abstand zu dem Strahl vorgesehenen Halterung (24) für eine Abstandsmesseinrichtung (26), die in einer Messstellung einen Laserstrahl (28) auf einen Durchtrittspunkt (20) des Behandlungsstrahls auf der Haut (16) gerichtet ist und den Abstand zu dem Durchtrittspunkt (20) misst,
- und einer Auswerteeinrichtung, die aus dem gemessenen Abstandswert den Abstand vom Fokus (22) des Behandlungsstrahls zu dem Durchtrittspunkt (20) berechnet.

2. Vorrichtung nach Anspruch 1, **dadurch gekennzeichnet, dass** die Abstandsmesseinrichtung den Abstand zu dem Laserpunkt nach dem Prinzip der Lasertriangulation misst.

3. Vorrichtung nach Anspruch 1 oder 2, **dadurch gekennzeichnet, dass** die Halterung (26) eine Schwenkachse (27) besitzt, die senkrecht zu dem Behandlungsstrahl (18) steht, und der Laserstrahl in jeder Stellung der Halterung den Behandlungsstrahl (18) schneidet.

4. Vorrichtung nach Anspruch 1 oder 2, **dadurch gekennzeichnet, daß** die Anordnung von Laser und Sensor nach dem Prinzip der Off-axis Laser Triangulation erfolgt, bei dem eine konfokale Abstandsmessung bei Anbringung des Messsystems außerhalb des Behandlungsstrahls erfolgt.

5. Vorrichtung nach einem der Ansprüche 1 bis 4, **dadurch gekennzeichnet, dass** die Auswerteeinrichtung (30) bei Abweichung des gemessenen Abstands vom vorbestimmten Sollwert die Strahlungsquelle abschaltet.

6. Vorrichtung nach Anspruch 5, **dadurch gekennzeichnet, dass** der gemessene Abstandswert als Führungsgröße für eine Steuerung der Position des Patiententisches (34) dient.

7. Vorrichtung nach einem der Ansprüche 1 bis 6, **dadurch gekennzeichnet, dass** der gemessene Abstandswert als Führungsgröße für die Strahlungsintensität dient.
